(19) Europäisches Patentamt
European Patent Office
Office européen des brevets

(11) **EP 3 564 901 A1**

(12) **EUROPEAN PATENT APPLICATION**
published in accordance with Art. 153(4) EPC

(43) Date of publication:
06.11.2019 Bulletin 2019/45

(51) Int Cl.:
*G06T 7/00* (2017.01)  *A61B 1/045* (2006.01)
*A61B 8/08* (2006.01)

(21) Application number: 17888366.6

(22) Date of filing: 12.12.2017

(86) International application number:
PCT/JP2017/044527

(87) International publication number:
WO 2018/123559 (05.07.2018 Gazette 2018/27)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD TN

(30) Priority: 28.12.2016 JP 2016254752

(71) Applicant: Kyoto University
Kyoto-shi, Kyoto 606-8501 (JP)

(72) Inventors:
• NAKAO Megumi
  Kyoto-shi
  Kyoto 606-8501 (JP)
• MATSUDA Tetsuya
  Kyoto-shi
  Kyoto 606-8501 (JP)
• MORITA Mitsuki
  Kyoto-shi
  Kyoto 606-8501 (JP)

(74) Representative: Hoffmann Eitle
Patent- und Rechtsanwälte PartmbB
Arabellastraße 30
81925 München (DE)

(54) **INFORMATION PROCESSING DEVICE, INFORMATION PROCESSING METHOD, AND PROGRAM**

(57)    An information processing device includes a computation unit that minimizes a difference between an observed displacement and an estimated displacement of an elastic body so as to find an optimal solution that determines a magnitude of an external force that acts upon an observed manipulation point and a distribution of an elastic modulus in a three-dimensional model of the elastic body, wherein the observed displacement is that acquired from an observed part of the elastic body in an image, and the estimated displacement is that acquired from a region of the three-dimensional model of the elastic body which has been captured, the region corresponding to the observed part.

FIG.3

**Description**

Technical Field

The present invention relates to an information processing device, an information processing method and a program.

Background Art

As can be seen in cancerous lesion palpation, information about stiffness of a body tissue is useful for a doctor in making diagnosis. In recent years, an elastography technique quantitatively and objectively evaluating elasticity information of a body tissue, such as an organ, and conducting image display of the distribution thereof has been put into practical use. Examples of the elastography technique include the magnetic resonance elastography (MRE) and the ultrasonic elastography.

The MRE is an elastic modulus measurement method using the magnetic resonance imaging (MRI), which captures a measurement target in the state of being vibrated from the outside and images the vibrational wave propagating within the target substance. The ultrasonic elastography is one of methods which are represented by a method of utilizing a strain distribution in a tissue generated when an external pressure is applied to the measurement target, or a method of generating shear waves in a target tissue and utilizing a propagation rate thereof.

Citation List

Non-Patent Literature

Non-Patent Document 1
O. Goksel, H. Eskandari, and S. E. Salcudean, "Mesh adaptation for improving elasticity reconstruction using the FEM inverse problem" IEEE Trans. Med. Imaging, vol. 32, no. 2, pp. 408-418, 2013, p21-23

Summary of Invention

Technical Problem

Incidentally, a range of a region where the elasticity information can be acquired by the elastography technique is limited to a range in the vicinity of a local region where waves propagate (a surface portion of the target tissue) or a region where a pressure is applied.

Therefore, an object of the present invention is to provide a method of estimating a distribution of an elastic modulus in an entire elastic body based on local displacement information.

Solution to Problem

The invention described in claim 1 provides an information processing device including: a computation unit that determines, as an optimal solution which minimizes a difference between an observed displacement having been acquired from an image in which a portion of an elastic body has been captured and an estimated displacement of a partial region corresponding to an observation region from among estimated displacements calculated based on a three-dimensional model of the elastic body, a magnitude of an external force that acts upon an observed manipulation point and a distribution of an elastic modulus in the three-dimensional model.

The invention described in claim 2 provides the information processing device according to claim 1, wherein the computation unit determines the magnitude of the external force and the distribution of the elastic modulus based on an equation as follows:

$$E^* = \underset{E,f}{\mathrm{argmin}}\, J(E,f) = \underset{E,f}{\mathrm{argmin}}\{\|u_o - L_o(E)f\|_2 + \lambda\|E - E_0\|_1\}$$

where $E^*$ represents a set of $E$ and $f$ that minimizes an evaluation function $J(E, f)$; $E$ represents an elastic modulus; $E_0$ represents a reference value of the elastic modulus; $f$ represents a magnitude of an external force; $u_o$ represents an observed displacement; $L_o(E)$ represents an observed part of an inverse matrix component of a total stiffness matrix $K$ expressed by the elastic modulus $E$; $L_o(E)f$ represents an estimated displacement; $\lambda$ represents a coefficient; $\|\ \|_1$

represents a one-dimensional norm; and $|\ |\ |_2$ represents a two-dimensional norm.

The invention described in claim 3 provides an information processing device including: an acquisition unit that acquires an observed part of displacement from an image of an elastic body having been captured; an acceptance unit that accepts a manipulation point of an external force acting upon the elastic body; an estimation unit that estimates an estimated displacement of a three-dimensional model of the elastic body by using a distribution of an elastic modulus in the three-dimensional model and a magnitude of the external force as variables; and an optimization unit that minimizes a difference between the observed displacement and the estimated displacement corresponding to the observed displacement, so as to find an optimal solution that determines the magnitude of the external force and the distribution of the elastic modulus in the three-dimensional model.

The invention described in claim 4 provides the information processing device according to claim 3, wherein the optimization unit determines the magnitude of the external force and the distribution of the elastic modulus based on an equation as follows:

$$E^* = \underset{E,f}{\mathrm{argmin}}\, J(E,f) = \underset{E,f}{\mathrm{argmin}}\{\|u_o - L_o(E)f\|_2 + \lambda\|E - E_0\|_1\}$$

where $E^*$ represents a set of E and f that minimizes an evaluation function J (E, f); E represents an elastic modulus; Eo represents a reference value of the elastic modulus; f represents a magnitude of an external force; $u_o$ represents an observed displacement; $L_o(E)$ represents an observed part of an inverse matrix component of a total stiffness matrix K expressed by the elastic modulus E; $L_o(E)f$ represents an estimated displacement; $\lambda$ represents a coefficient; $|\ |\ |_1$ represents a one-dimensional norm; and $|\ |\ |_2$ represents a two-dimensional norm.

The invention described in claim 5 provides the information processing device according to claim 1 or 3, further including: an expression unit that expresses the three-dimensional model based on the distribution of the elastic modulus determined as the optimal solution.

The invention described in claim 6 provides the information processing device according to claim 1 or 3, wherein the image is a still image captured by a single image-capturing camera.

The invention described in claim 7 provides the information processing device according to claim 1 or 3, wherein the optimal solution is determined as a value that minimizes a sum total of a sum of absolute values of changings from an initial value of the distribution of the elastic modulus and the difference.

The invention described in claim 8 provides the information processing device according to claim 1 or 3, wherein, when plural pieces of information about the manipulation point are provided, the optimal solution is determined as a value that minimizes a sum total of the differences calculated for the individual manipulation points.

The invention described in claim 9 provides the information processing device according to claim 1 or 3, wherein, when the plural external forces are applied to the manipulation point, the optimal solution is determined as a value that minimizes a sum total of the differences calculated for the individual external forces.

The invention described in claim 10 provides the information processing device according to claim 1 or 3, wherein there are the plural manipulation points, and the external forces, which are different from one another, are applied to the respective manipulation points.

The invention described in claim 11 provides the information processing device according to claim 1 or 3, wherein a spatial resolution that determines the optimal solution is gradually increased from a low level.

The invention described in claim 12 provides the information processing device according to claim 1 or 3, wherein, when a magnitude of the external force that acts upon the manipulation point is given, the optimal solution is determined as the distribution of the elastic modulus that minimizes the difference.

The invention described in claim 13 provides an information processing method including: a process that acquires an observed part of displacement from an image of an elastic body having been captured; a process that accepts a manipulation point of an external force acting upon the elastic body; a process that estimates an estimated displacement of a three-dimensional model of the elastic body by using a distribution of an elastic modulus in the three-dimensional model and a magnitude of the external force as variables; and a process that minimizes a difference between the observed displacement and the estimated displacement corresponding to the observed displacement, so as to find an optimal solution that determines the magnitude of the external force and the distribution of the elastic modulus in the three-dimensional model.

The invention described in claim 14 provides a program causing a computer to execute: a process that acquires an observed part of displacement from an image of an elastic body having been captured; a process that accepts a manipulation point of an external force acting upon the elastic body; a process that estimates an estimated displacement of a three-dimensional model of the elastic body by using a distribution of an elastic modulus in the three-dimensional model and a magnitude of the external force as variables; and a process that minimizes a difference between the observed displacement and the estimated displacement corresponding to the observed displacement, so as to find an optimal solution that determines the magnitude of the external force and the distribution of the elastic modulus in the three-dimensional model.

Advantageous Effects of Invention

According to the invention described in claim 1, even when the region where the displacement can be acquired is limited to a part of the elastic body, it is possible to determine the distribution of the elastic modulus in the entire region of the elastic body.

According to the invention described in claim 2, even when the region where the displacement can be acquired is limited to a part of the elastic body, it is possible to determine the distribution of the elastic modulus in the entire region of the elastic body.

According to the invention described in claim 3, even when the region where the displacement can be acquired is limited to a part of the elastic body, it is possible to determine the distribution of the elastic modulus in the entire region of the elastic body.

According to the invention described in claim 4, even when the region where the displacement can be acquired is limited to a part of the elastic body, it is possible to determine the distribution of the elastic modulus in the entire region of the elastic body.

According to the invention described in claim 5, it is possible to visually confirm the distribution of the elastic modulus on the three-dimensional model.

According to the invention described in claim 6, it is possible to simplify a device configuration used for observation of the elastic body.

According to the invention described in claim 7, it is possible to determine the distribution of the elastic modulus that is highly localized.

According to the invention described in claim 8, it is possible to increase estimation accuracy as compared to a case including only a single manipulation point of the external force used for acquiring the displacement.

According to the invention described in claim 9, it is possible to increase estimation accuracy as compared to a case including only a single external force used for acquiring the displacement.

According to the invention described in claim 10, it is possible to increase estimation accuracy as compared to a case including only one manipulation point and only one external force used for acquiring the displacement.

According to the invention described in claim 11, it is possible to obtain an estimation result with higher accuracy as time passes.

According to the invention described in claim 12, it is possible to increase estimation accuracy as compared to a case in which a magnitude of the external force is not provided.

According to the invention described in claim 13, even when the region where the displacement can be acquired is limited to a part of the elastic body, it is possible to determine the distribution of the elastic modulus in the entire region of the elastic body.

According to the invention described in claim 14, even when the region where the displacement can be acquired is limited to a part of the elastic body, it is possible to determine the distribution of the elastic modulus in the entire region of the elastic body.

Brief Description of Drawings

FIG. 1 is a diagram illustrating a concept image of the invention described in exemplary embodiments;
FIG. 2 is a diagram showing a hardware configuration example in an information processing system;
FIG. 3 is a diagram illustrating a functional configuration example accomplished through execution of a computer program;
FIG. 4 is a diagram showing an example of a mesh model of an elastic body;
FIG. 5 is a diagram illustrating a deformation example of the mesh model of the elastic body;
FIG. 6 is a diagram showing a two-dimensional mesh model;
FIG. 7 is a diagram illustrating a concept of superimposition processing of an element stiffness matrix Ke;
FIG. 8 is a diagram showing a distribution example of an elastic modulus E providing a true value in a simulation experiment;
FIG. 9 is a diagram illustrating a ratio of an observed part;
FIG. 10 is a diagram illustrating a relationship between the number of estimation blocks and a size of a unit section (block) for which the elastic modulus E is estimated;
FIG. 11 is a diagram showing experimental results in the cases where the number of estimation blocks N is 10, 20, and 40;

FIG. 12 is a diagram illustrating observation conditions used in Exemplary embodiment 2;

FIG. 13 is a diagram showing experimental results in the cases of using one set (Exemplary embodiment 1) and two sets (Exemplary embodiment 2) of patterns for estimating the distribution of the elastic modulus E;

FIG. 14 is a diagram showing experimental results in the cases of using one set (Exemplary embodiment 1) and two sets (Exemplary embodiment 2) of patterns for estimating the distribution of elastic modulus E, and using sparsity of changes in the elastic modulus E in addition to the case of two sets of patterns (Exemplary embodiment 3); and

FIG. 15 is a diagram illustrating a relationship between the ratio of the number of observed vertexes and the observation accuracy in the case of Exemplary embodiment 3.

Description of Embodiments

Hereinafter, with reference to attached drawings, exemplary embodiments according to the present invention will be described in detail.

<Concept image>

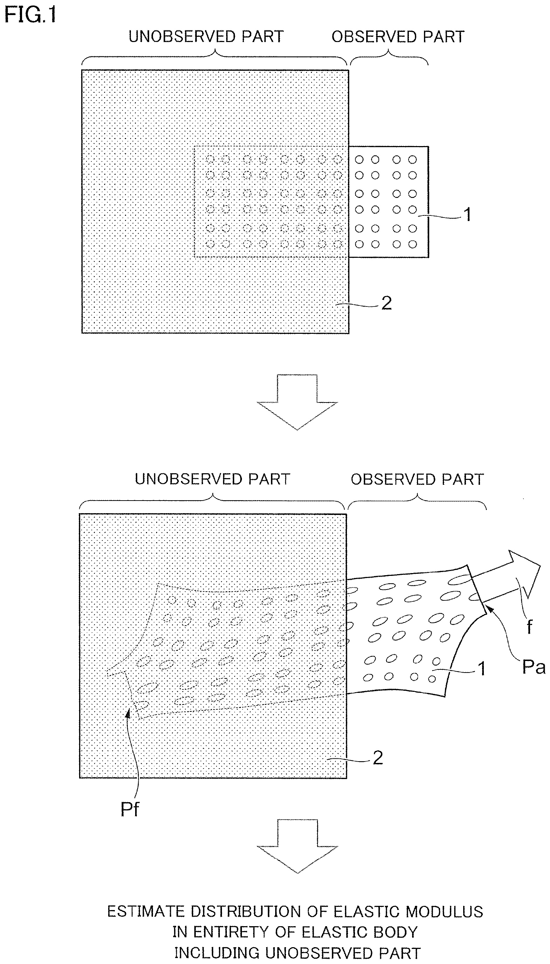

FIG. 1 is a diagram illustrating a concept image of an estimation method adopted in the exemplary embodiments to be described later. As shown in FIG. 1, in each exemplary embodiment, a case where the entirety of an elastic body 1 cannot be observed (in other words, a case where there are observed parts and unobserved parts) is regarded as a precondition, and in each exemplary embodiment, description will be given of a method of estimating a distribution of an elastic modulus in the entire elastic body by use of local displacement information in the observed part.

In each exemplary embodiment, the estimation processing is executed by using the fact that positional information of a manipulation point Pa of an external force F that deforms the elastic body 1 is observed.

The positional information of the manipulation point Pa may be provided to the processing system before the estimation processing is started, and the position of the manipulation point Pa is arbitrary. Consequently, as shown in FIG. 1, it is unnecessary that the position of the manipulation point Pa exists in the observed part.

There is no limit on the number of manipulation points Pa; accordingly, the number is not necessarily one. Consequently, the number of the manipulation points Pa may be one, plural, or the manipulation points Pa may be provided on the entire surface of the elastic body 1.

On the other hand, information providing a magnitude f of the external force F that acts upon the manipulation point Pa or positional information of a point where the elastic body 1 is fixed to the surroundings (a fixed point) Pf may be observed or unobserved. In general, the more the observed pieces of information, the higher the estimation accuracy becomes, and therefore, the calculation time required for estimation is reduced.

In FIG. 1, to describe the observed part and the unobserved part, the state in which a part of the elastic body 1 is covered with a shield 2 is shown; however, this does not mean that a part of the elastic body 1 is necessarily covered with the shield 2. For example, when the elastic body 1 is observed from only one direction, a part included in an observation field is the observed part, and a part not included in the observation field is the unobserved part. Consequently, a part which is an unobserved region of the elastic body 1 (for example, a reverse side or a back surface side) is an example of the unobserved part.

Moreover, in the exemplary embodiments to be described later, the displacement in the observed part of the elastic body 1 is assumed to be identified for each part. In other words, it is assumed that the displacement in the elastic body 1 due to application of the external force F is provided to the processing system as observed information.

Depending on the difference in observation units, the displacement is provided only to a surface region of the elastic body 1 in some cases, and is provided to the elastic body 1 including the inner regions thereof in other cases. In general, the more the regions where the displacement is identified (as the ratio of the observed parts is increased), the higher the estimation accuracy of the elastic modulus becomes.

<Terms>

The elastic modulus in each exemplary embodiment includes: Young's modulus E representing stiffness in pressing or pulling directions; a stiffness modulus G representing stiffness in a shear direction; Poisson's ratio v; a volume elastic modulus K; and the like, and, in the exemplary embodiments to be described later, the elastic modulus is used on the assumption that the elastic modulus is Young's modulus.

In each exemplary embodiment, the elastic body 1 refers to a substance having a property of, while generating strain (being deformed) by application of an external force F, returning to an original dimension when application of the external force F is stopped. In the exemplary embodiment, an elastic body represented by approximately using Hooke's law, such as, for example, various kinds of organs in tissues from a living body, is assumed.

However, in each exemplary embodiment, a case in which the distribution of the elastic modulus in the elastic body 1 is not uniform is assumed. Here, the case in which the distribution of the elastic modulus is not uniform means that, for example, the elastic modulus of at least a part of the elastic body 1 is different from the elastic modulus of the surrounding parts.

<Exemplary embodiment 1>

<System configuration

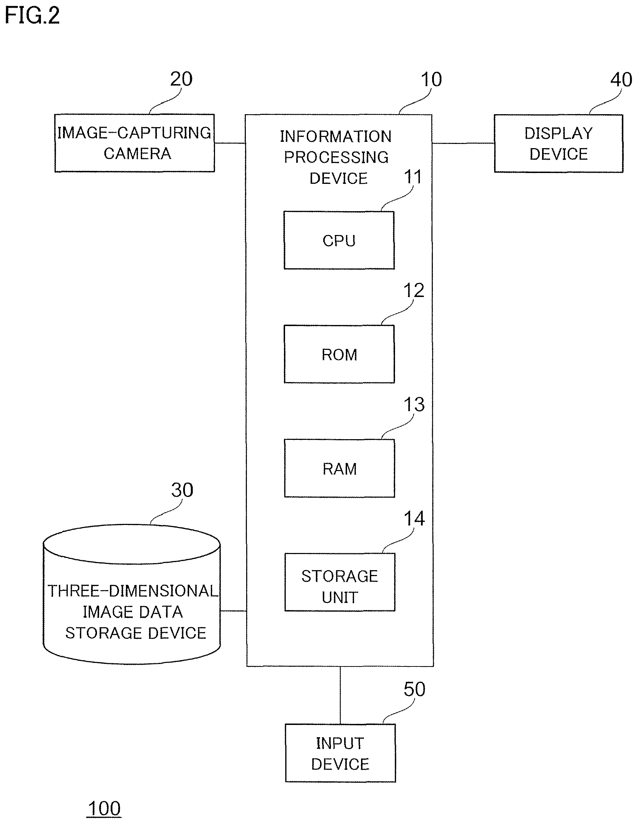

FIG. 2 is a diagram showing a configuration example of hardware in an information processing system 100.
The information processing system 100 includes: an information processing device 10 that executes arithmetic processing for estimating the distribution of the elastic modulus in the elastic body 1 (refer to FIG. 1); at least one image-capturing camera 20 used for capturing images of the elastic body 1; a three-dimensional image data storage device 30 that stores three-dimensional image data (volume data) used for generating a mesh model of the elastic body 1; a display device 40 used for displaying the estimated distribution of the elastic modulus; and an input device 50 configured with a mouse, a pointing device and the like operated by a user.
The information processing device 10 is a so-called computer and configured with: a CPU (central processing unit) 11 that executes programs; a ROM (read only memory) 12 that stores programs, such as BIOS (basic input/output system) or firmware, or data; a RAM (random access memory) 13 that provides programs with work areas; and a storage unit 14 that stores image data to be subjected to processing. The storage unit 14 is a storage device, such as a hard disk device or a semiconductor memory. The functions accomplished through execution of programs by the CPU 11 will be described later.
The image-capturing camera 20 is an image-capturing device used for observation of the elastic body 1. The image-capturing camera 20 may be an image-capturing device suitable for observation of the elastic body 1. For example, as the image-capturing camera 20, an infrared camera, an endoscopic camera, an ultrasonic imaging device, a photoacoustic imaging device or the like is used.
When the infrared camera or the endoscopic camera is used, the information processing device 10 can identify the displacement in each region via the movement of the feature point or infrared marker existing on the surface of the elastic body 1. When the ultrasonic imaging device is used, the information processing device 10 can identify the displacement in each region including the interiors of the elastic body 1. Note that an imaging element of the endoscopic camera may be a CCD (Charge Coupled Device) or a CMOS (Complementary MOS).
In the exemplary embodiment, a single image-capturing camera 20 is used to output a still image in which the elastic body 1 has been captured from one direction.
The three-dimensional image data storage device 30 is configured with a large-capacity hard disk device or the like that stores, as three-dimensional image data, an image reconfigured (a reconfiguration image) by accumulating pieces of tomographic image data acquired by taking tomography of the elastic body 1.
The three-dimensional image data storage device 30 can, not only be directly connected to the information processing device 10, but also exist on a network as a server or a network storage. Here, for acquiring the tomographic image data, for example, CT (Computed Tomography), PET (Positron Emission Tomography), MRI or the like is used.
The display device 40 is configured with, for example, a liquid crystal display. The liquid crystal display is configured with a liquid crystal panel, a backlight, and so forth. Note that the display device 40 may be an organic EL (Electroluminescence) display or the like.

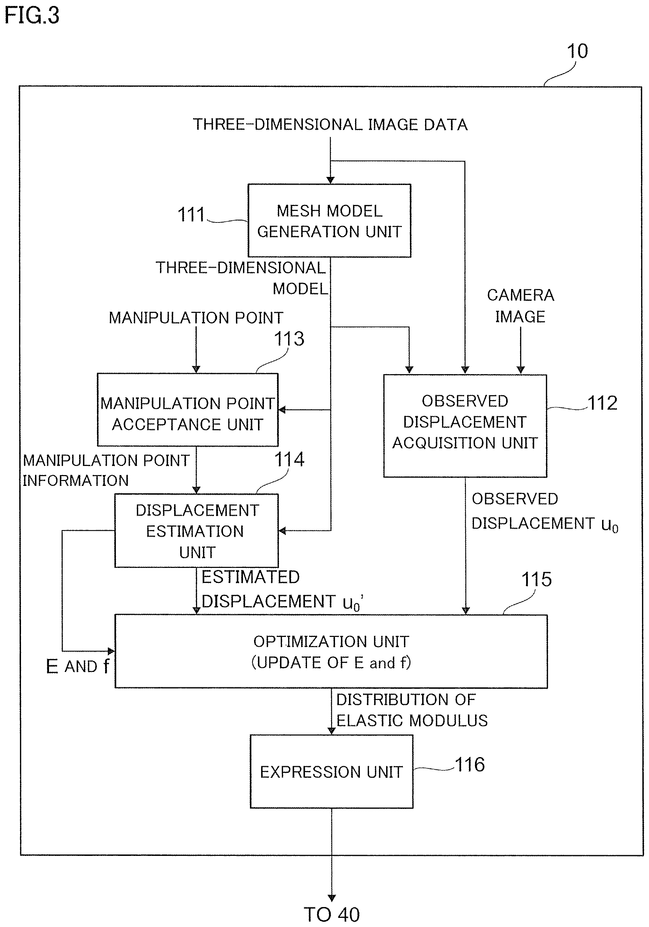

FIG. 3 is a diagram illustrating a functional configuration example accomplished through execution of computer programs by the CPU 11.
The information processing device 10 from the standpoint of the processing functions includes: a mesh model generation unit 111 that generates a three-dimensional mesh model; an observed displacement acquisition unit 112 that acquires the displacement of the observation region (an observed displacement $u_o$) from a camera image outputted by the image-capturing camera 20; a manipulation point acceptance unit 113 that accepts positional information of the manipulation point Pa; a displacement estimation unit 114 that calculates the displacement in each point of the mesh model (an estimated displacement $u_o'$) by applying manipulation point information to the mesh model; an optimization unit 115 that minimizes a difference between the estimated displacement $u_o'$ and the observed displacement $u_o$ so as to find an optimal solution that determines the elastic modulus E (the element stiffness matrix K being a function of the elastic modulus E as a result) and the magnitude f of the external force F; and an expression unit 116 that generates an image expressing superimposition of the distribution of the elastic modulus identified by the element stiffness matrix K regarded as the optimal solution on the mesh model and outputs thereof to the display device 40. These functional units are an example of "a computation unit" in claims.
The mesh model generation unit 111 is a functional unit that takes in the three-dimensional image data of the elastic body 1 regarded as an observation object from the three-dimensional image data storage device 30, and then generates a mesh model, which is an aggregation of simple shape elements, from the three-dimensional image data provided as a continuous body. The mesh model here is an example of the three-dimensional model, and a minimum element thereof is expressed by a cube or a tetrahedron (triangular pyramid).

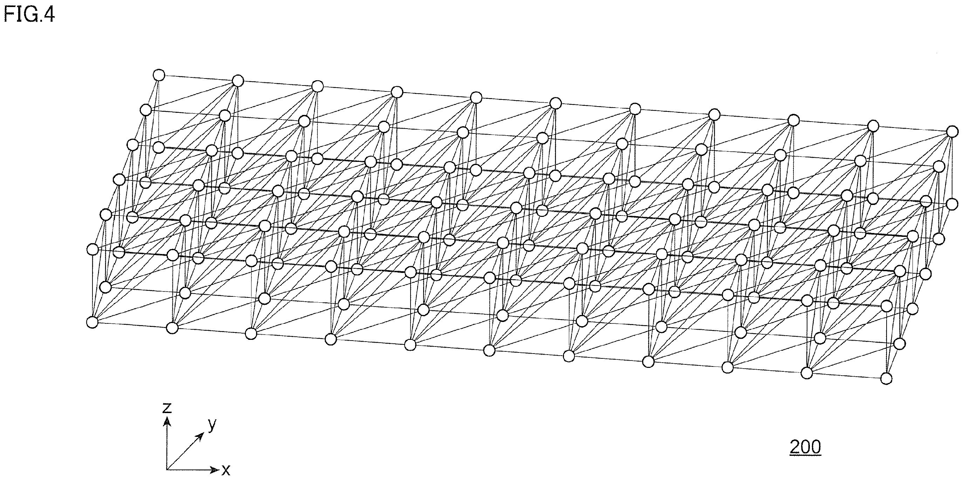

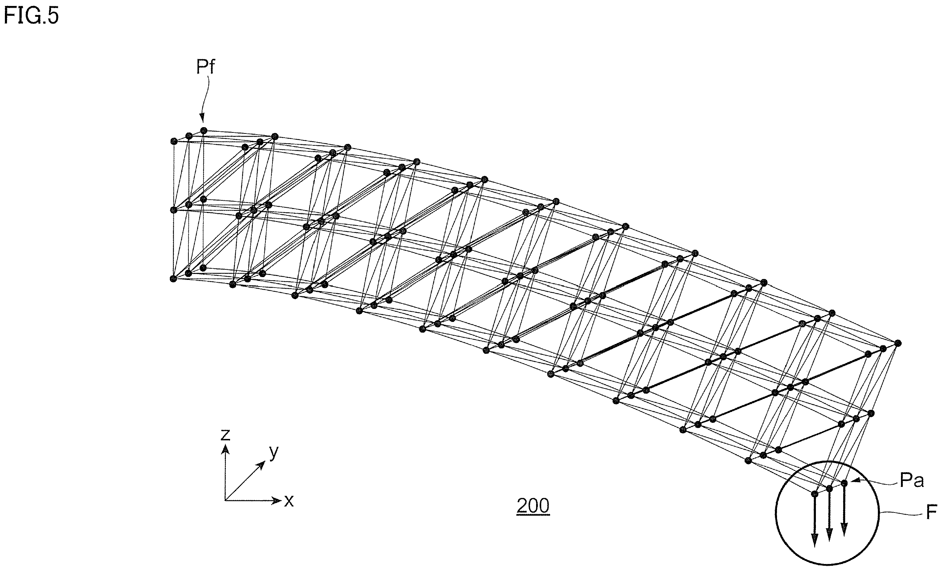

FIG. 4 is a diagram showing an example of a mesh model 200 expressing the elastic body 1 by a mesh structure. FIG. 4 depicts the mesh model 200 before the external force F (refer to FIG. 5) is applied to the manipulation point Pa (refer to FIG. 5). Circles in the figure depict nodes of the minimum elements. Note that FIG. 5 depicts the mesh model 200 elastically deformed by application of the external force F to the lower right end part of the mesh model 200 as the manipulation point Pa.

The observed displacement acquisition unit 112 is a functional unit that compares the camera image before the external force F is applied to the manipulation point Pa with the camera image after the external force F is applied to the manipulation point Pa to acquire the observed displacement $u_o$ in each region appearing in the image.

Note that the observed displacement $u_o$ acquired in the observed displacement acquisition unit 112 is the displacement of each position identified in the camera image, and positional relationship thereof with voxels constituting the mesh model 200 is uncertain.

Therefore, the observed displacement acquisition unit 112 is provided with a processing function that matches the three-dimensional image data or the mesh model 200 (refer to FIG. 4) with the camera image, and associates the observed displacement $u_o$ with each vertex of the mesh model 200. When the association is performed here, processing for aligning the dimension of the three-dimensional image data or the mesh model 200 and the dimension of the camera image is also executed.

In general, the more pieces of image information are included in the three-dimensional image data or the mesh model 200, the higher the matching accuracy with the camera image becomes. For example, when the mesh model 200 is used for matching, by comparing the rendering image formed by rendering the surface information of the three-dimensional image data on the surface of the mesh model 200 with brightness information of the camera image, it is possible to associate the observed displacements $u_o$ with the vertexes of the mesh model 200 with high matching accuracy.

The manipulation point acceptance unit 113 is a functional unit that accepts the positional information of the manipulation point Pa through the input device 50. The manipulation point acceptance unit 113 accepts, for example, when clicking is detected, the point on the mesh model 200 where a mouse cursor is positioned as the manipulation point Pa of the external force F.

The manipulation point acceptance unit 113 identifies the position of the accepted manipulation point Pa by the identification number of the vertex on the mesh model 200, and outputs thereof to the displacement estimation unit 114.

The displacement estimation unit 114 is a functional unit that calculates the estimated displacements $u_o$' for all the vertexes constituting the mesh model 200 by use of the equation of equilibrium of force used in the linear finite element method (in other words, an equation expressing Hooke's law).

Specifically, the displacement estimation unit 114 calculates the estimated displacement $u_o$' based on Equation 1 which is the modification of the equation of equilibrium of force ($f = K \cdot u_o$'), where f is the magnitude of the external force F applied to each vertex of the mesh model 200, $u_o$' is the estimated displacement on each vertex, and K is the total stiffness matrix.

$$\boldsymbol{u}_o' = K^{-1}\boldsymbol{f} = L\boldsymbol{f} \qquad \text{... Equation 1}$$

Note that the index o means a vertex positioned at an observed part, and L means an inverse matrix ($K^{-1}$) of the total stiffness matrix K.

In the exemplary embodiment, the distribution of the elastic modulus E of the elastic body 1 and the magnitude f (the vector) of the external force F applied to the elastic body 1 are the final estimation targets. Note that, since the elastic modulus E is an intensional parameter of the total stiffness matrix K, if the total stiffness matrix K is identified, the elastic modulus E is identified, too.

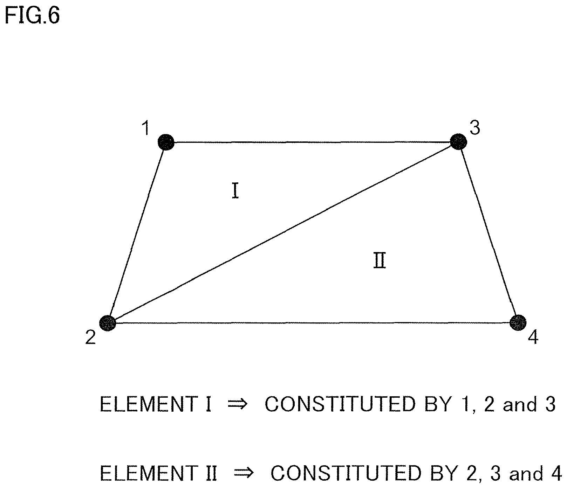

Hereinafter, to simplify the description, the relationship between the total stiffness matrix K and the distribution of the elastic modulus E will be described by using FIG. 6. FIG. 6 shows a two-dimensional mesh model. Needless to say, the information processing system 100 handles the three-dimensional mesh model 200.

In the case of the mesh model shown in FIG. 6, the triangular element I is defined by vertex numbers 1, 2 and 3, and the triangular element II is defined by vertex numbers 2, 3 and 4. At this time, the force $f_i$ that acts upon the vertex number i (i = 1 to 4) and the displacement $u_i$ at the vertex number i (i = 1 to 4) are given as the equation of equilibrium of force in the linear finite element method using the total stiffness matrix K (Equation 2).

$$\begin{pmatrix} f_1 \\ f_2 \\ f_3 \\ f_4 \end{pmatrix} = \begin{bmatrix} & K & \end{bmatrix} \begin{pmatrix} u_1 \\ u_2 \\ u_3 \\ u_4 \end{pmatrix}$$

... Equation 2

Here, the element stiffness matrix $K_e$ used for association of the magnitude $f_i$ of the external force F that acts upon each vertex number i (i = 1 to 4) with the displacement $u_i$ thereof is derived by the following Equation 3.

$$K_e = V B^T D^T B$$

... Equation 3

Here, V represents a volume of the triangular element.

Moreover, B matrix is determined from a shape function of an element, and D matrix is determined by the elastic modulus E and Poisson's ratio v.

The D matrix can be expressed by the following equation (Equation 4).

$$D = \frac{E}{(1+v)(1-2v)} \begin{bmatrix} 1-v & v & v & 0 & 0 & 0 \\ v & 1-v & v & 0 & 0 & 0 \\ v & v & 1-v & 0 & 0 & 0 \\ 0 & 0 & 0 & \dfrac{1-2v}{2} & 0 & 0 \\ 0 & 0 & 0 & 0 & \dfrac{1-2v}{2} & 0 \\ 0 & 0 & 0 & 0 & 0 & \dfrac{1-2v}{2} \end{bmatrix}$$

... Equation 4

As can be seen from Equation 4, the D matrix is a function of the elastic modulus E. Consequently, the element stiffness matrix $K_e$ is also expressed as the function of the elastic modulus E.

The total stiffness matrix K is given by a sum of the element stiffness matrix $K_e$ of the triangular element I and the element stiffness matrix $K_e$ of the triangular element II. In other words, the total stiffness matrix K is given by a sum of the element stiffness matrix $K_e$ of the triangular element I constituted by the vertex numbers 1, 2 and 3 and the element stiffness matrix $K_e$ of the element stiffness matrix II constituted by the vertex numbers 2, 3 and 4.

As shown in FIG. 6, in the triangular element I and the triangular element II, there are some vertexes overlapping each other. Therefore, superimposition of the element stiffness matrix $K_e$ is performed for each overlapping vertex.

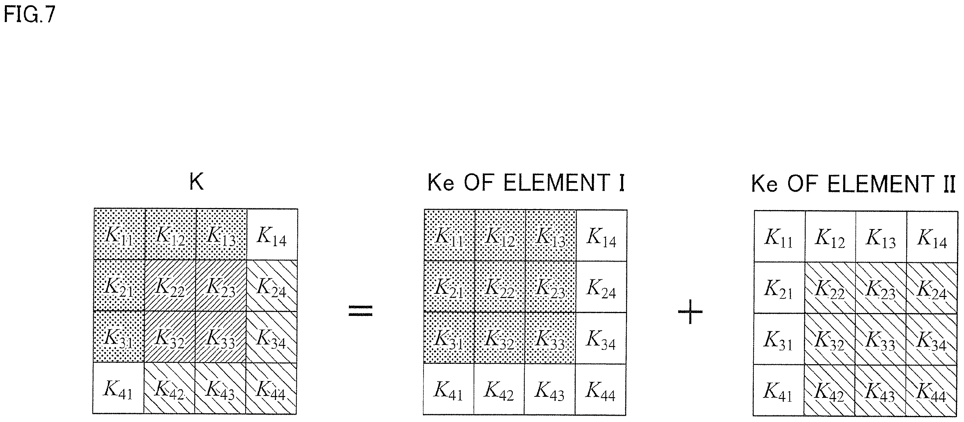

FIG. 7 is a diagram illustrating a concept of superimposition processing of the element stiffness matrix $K_e$. FIG. 7 shows that the total stiffness matrix K is expressed by a sum of the element stiffness matrix $K_e$ of the element I and the element stiffness matrix $K_e$ of the element II. As described above, since the element stiffness matrix $K_e$ is a function of the elastic modulus E, the total stiffness matrix K expressed by the sum of them is also a function of the elastic modulus E.

Consequently, the total stiffness matrix K in the mesh model 200 of the exemplary embodiment is also a function of the elastic modulus E.

By the way, as described above, the camera image in the exemplary embodiment is an image in which a part of the elastic body 1 elastically deformed by the external force F has been captured, the manipulation point Pa thereof being observed but the magnitude f thereof being unobserved.

Consequently, unobserved parameters of Equation 2 are the distribution of the elastic modulus E (the total stiffness matrix K) and the magnitude f (the vector) of the external force F.

Therefore, the displacement estimation unit 114 sets arbitrary values for these respective unobserved parameters to calculate the estimated displacements $u_o{}'$. Note that, in the first estimation of the estimated displacement $u_o{}'$, the displacement estimation unit 114 provides an initial value to each of the distribution of the elastic modulus E (the total stiffness matrix K) and the magnitude f of the external force F, which are the unobserved parameters, to calculate the estimated displacement $u_o{}'$.

Expression of Equation 2 by a matrix results in the following equation (Equation 5).

$$\begin{bmatrix} u_o' \\ \hline u_i' \end{bmatrix} = \begin{bmatrix} L_o \\ \hline L_i \end{bmatrix} \begin{bmatrix} f_o \\ \hline f_i \end{bmatrix} \qquad \text{... Equation 5}$$

Here, $u_o'$ is the estimated displacement in the observed part, and $u_i'$ is the estimated displacement in the unobserved part. Moreover, $L_o$ is the observed part of the inverse matrix component of the total stiffness matrix K expressed by the elastic modulus E, and $L_i$ is the unobserved part of the inverse matrix component of the total stiffness matrix K expressed by the elastic modulus E. Further, $f_o$ is the magnitude of the external force F that acts upon the vertex in the observed part, and $f_i$ is the magnitude of the external force F that acts upon the vertex in the unobserved part.

Focusing on the estimated displacement $u_o'$ of the observed part, Equation 5 can be expressed as the following equation (Equation 6).

$$\begin{bmatrix} u_o' \end{bmatrix} = \begin{bmatrix} L_o \end{bmatrix} \begin{bmatrix} f_o \\ \hline f_i \end{bmatrix} = \begin{bmatrix} L_o \end{bmatrix} \begin{bmatrix} f \end{bmatrix} \qquad \text{... Equation 6}$$

Here, since the manipulation point Pa has already been observed, of the magnitudes f of the external forces F, the magnitudes of the external forces F corresponding to the vertexes other than the manipulation point Pa can be expressed by 0 (zero).

The displacement estimation unit 114 calculates the estimated displacement $u_o'$ (including the initial value) from the component $L_o$ expressed by the elastic modulus E and the magnitude f of the external force F by use of Equation 6.

The optimization unit 115 updates combinations of the unobserved parameters until the total stiffness matrix K (in other words, the distribution of the elastic modulus E) and the external force f to minimize the difference between the estimated displacements $u_o'$ estimated for all the vertex numbers of the mesh model 200 (including not only the observed parts, but also the unobserved parts) and the observed displacements $u_o$ identified by the partial images captured by the image-capturing camera 20.

Specifically, the optimization unit 115 executes processing for solving the optimization problem that determines the unobserved parameter to minimize the difference between the estimated displacement $u_o'$ and the observed displacement $u_o$ (that is, to provide the deformation most approximate to the observation result). Expression of the processing results in the following equation (Equation 7).

$$E^* = \underset{E,f}{\operatorname{argmin}} J(E, f) = \underset{E,f}{\operatorname{argmin}} \| u_o - u_o' \|_2$$

$$= \underset{E,f}{\operatorname{argmin}} \| u_o - L_o(E)f \|_2 \qquad \text{... Equation 7}$$

Here, $E^*$ means a set of E and f that minimizes the evaluation function J (E, f).

In the exemplary embodiment, the evaluation function J (E, f) is given by an $L^2$ norm of the difference between the estimated displacement $u_o'$ and the observed displacement $u_o$.

The optimization unit 115 updates the unobserved parameters E and f until the minimum value of the evaluation function J is determined, and provides feedback about the updated value to the displacement estimation unit 114. In other words, recalculation of the estimated displacement $u_o'$ by the displacement estimation unit 114 by use of the updated values of the unobserved parameters E and f and evaluation of the estimated displacement $u_o'$ by the optimization unit 115 are repeatedly executed until the minimum value of the evaluation function J is determined.

In the exemplary embodiment, as a method of solving the optimization problem, the covariance matrix adaptation evolution strategy (CMA-ES) was used. However, as the method of solving the optimization problem, any other known methods may be used. For example, a gradient method, Monte Carlo method, simulated annealing, or the like may be used.

Note that, in FIG. 3, the displacement estimation unit 114 and the optimization unit 115 are depicted as different functional units; however, it may be possible to include thereof in one functional unit.

The expression unit 116 (refer to FIG. 3) displays an image expressing, of the distribution of the elastic modulus E and the magnitude of the external force F identified as the optimal solution, the distribution of the elastic modulus E superimposed on the mesh model 200 on the display device 40 (refer to FIG. 2). Due to the distribution of the elastic modulus E being expressed in association with the mesh model 200, a user can visually recognize the distribution of the elastic modulus E of the elastic body 1 including the unobserved parts.

Note that the expression unit 116 expresses the differences in the elastic modulus E as the differences in colors or brightness. Moreover, the expression unit 116 may display the numerical value of the elastic modulus E corresponding to each part of the mesh model 200. Note that the expression unit 116 outputs the magnitude f of the external force F determined as the optimal solution as needed.

<Verification by simulation experiment

Hereinafter, description will be given of a verification result of accuracy in the distribution of the elastic modulus E estimated by use of the mesh model 200 generated for the elastic body 1 (that is, the mesh model shown in FIG. 4), the observed displacement $u_o$ obtained by locally observing the elastic body 1 and information about the manipulation point Pa of the external force F that acts upon the elastic body 1.

Hereinafter, in the simulation experiment, it is assumed that the mesh model 200 corresponding to the elastic body 1 includes N vertexes, and description will be given of the effects of the number of observed vertexes on the estimation accuracy when the distribution of the elastic modulus E of the entire elastic body 1 is estimated by use of the observed displacements $u_o$ of the M vertexes (N > M).

In the simulation experiment, the method related to the exemplary embodiment was described as a program by use of Visual C/C++, and the generated program was executed by a general-purpose computer (OS: Windows 10 Pro, CPU: IntelCorei7-6700K, Memory: 16GB).

Note that the simulation was executed for the mesh model 200 in the shape of a rectangular parallelepiped (refer to FIG. 5). The mesh model 200 includes 99 vertexes in total, and the minimum element thereof is tetrahedron.

Moreover, as shown in FIG. 5, nine vertexes positioned on the left end of the mesh model 200 were set as the fixed end Pf, and the other vertexes were regarded as free points.

Further, as shown in FIG. 5, as the manipulation points Pa of the external forces F, three vertexes positioned on the lower right end part of the mesh model 200 were specified. It was assumed that, upon each of the three vertexes, the external force F of the same magnitude f acted in the downward direction (in the -z axis direction).

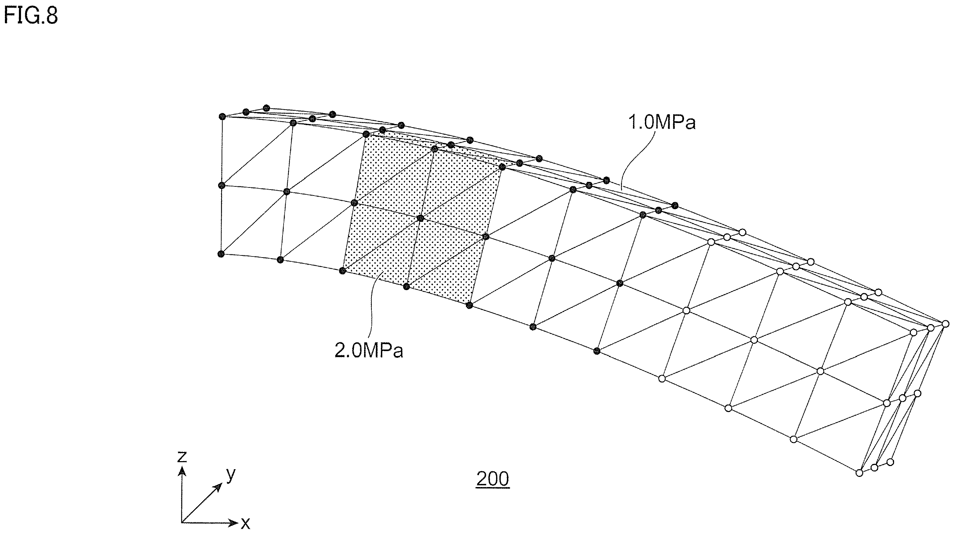

The deformation of the mesh model 200 by the external force F depends on the state of distribution of the elastic modulus E providing an internal structure of the elastic body 1. Here, description will be given of the internal structure as a true value used for evaluating the estimation accuracy in the distribution of the elastic modulus E estimated by the method suggested in the exemplary embodiment. FIG. 8 is a diagram depicting the distribution of the elastic modulus E used as a true value superimposed on the mesh model 200. In the mesh model 200 shown in FIG. 8, only the elastic modulus E of a part thereof is large (hard), and the elastic moduli E of the other parts have the same value. In the figure, the node of the white dot represents that the node is positioned in the observed part, and the node of the black dot represents that the node is positioned in the unobserved part.

The mesh model 200 shown in FIG. 8 represents a case in which, of the 40 cubic blocks, the elastic modulus E of the four cubic blocks, which are positioned at the seventh and eighth in the left row from the right end to the left direction (the -x direction), is as hard as 2.0 [MPa], and the elastic modulus E of the other parts is 1.0 [MPa].

In the simulation experiment, based on the camera image captured when the external force F was applied to the mesh model 200 shown in FIG. 8 and the positions of the manipulation points Pa, the distribution of the elastic moduli E of all the vertexes of the mesh model 200 was estimated.

Note that, similar to the description in FIG. 5, the mesh model 200 shown in FIG. 8 depicts the state in which the external force F of 10N was applied to the three vertexes at the lower right end part of the mesh model 200 of the elastic body 1 in the downward direction (in the -z axis direction) to deform the mesh model 200. Of course, for the information processing device 10, the magnitude f of the external force F is also an unobserved parameter.

As described above, the purpose of the simulation experiment is to evaluate the effects of the number of observed vertexes on the estimation accuracy of the elastic modulus E.

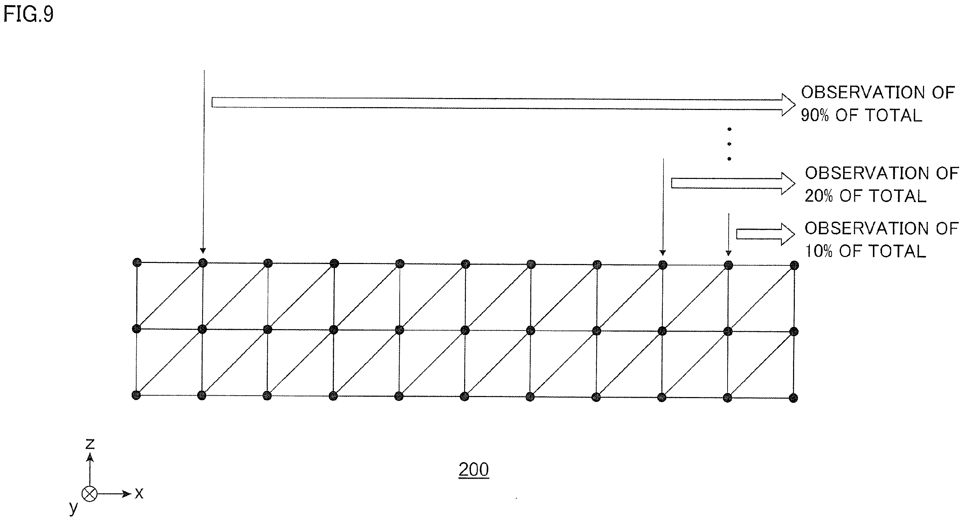

Therefore, in the simulation experiment, the cases in which the number of observed vertexes (the number of vertexes provided with the observed displacements $u_o$) accounts for 10%, 20%, ..., 90% of the total were evaluated. FIG. 9 is a diagram illustrating a ratio of the number of observed vertexes in the mesh model 200. FIG. 9 is a diagram observing the mesh model 200 from the lateral direction (on the x-z plane), where the mesh model 200 is divided into 10 small sections in the x axis direction. Of these, the case in which one small section is observed corresponds to the case in which 10% of the total is observed, and the case in which two small sections are observed corresponds to the case in which 20% of the total is observed. Hereinafter, similarly, the case in which nine small sections are observed corresponds to the case in which 90% of the total is observed.

Incidentally, it is expected that the estimation accuracy of the elastic modulus E is affected by the number of dimensions of the estimation space. Here, the number of dimensions is expressed by an estimated resolution of the elastic modulus E. For example, the small number of dimensions of the estimation space means that the estimation is performed at a low resolution, and the large number of dimensions means that the estimation is performed at a high resolution. To put it another way, the number of dimensions of the estimation space represents the number of unit sections (blocks) into which one mesh model 200 is divided.

In the following simulation experiment, the cases in which the number of estimation blocks N representing the estimated resolution of the elastic modulus E is 10, N = 20, and N = 40 were studied.

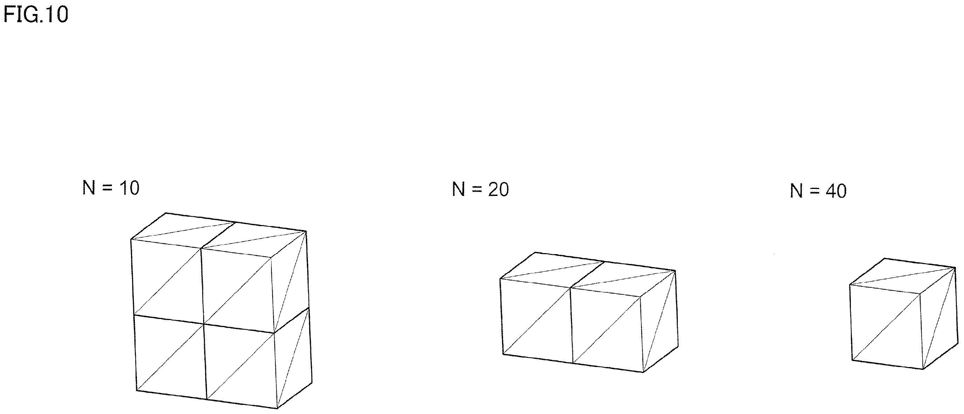

FIG. 10 is a diagram illustrating a relationship between the number of estimation blocks N and a size of a unit section (block) for which the elastic modulus E is estimated.

For example, the case of N = 10 means that the mesh model 200 (refer to FIG. 9) is expressed by 10 unit sections (blocks), the case of N = 20 means that the mesh model 200 is expressed by 20 unit sections (blocks), and the case of N = 40 means that the mesh model 200 is expressed by 40 unit sections (blocks).

In other words, the unit section (block) in the case of N = 40 has the same shape as the cubic block illustrated in FIG. 8. Naturally, the larger the value of the number of estimation blocks N (the smaller the size of the estimated unit section), the higher the spatial resolution becomes; therefore, the distribution of the elastic modulus E can be estimated with high definition.

Subsequently, description will be given of a relationship between the number of estimation blocks N and the ratio of the number of observation points required for obtaining high estimation accuracy. As will be described later, as the number of estimation blocks N is increased (as the size of the estimated unit section is reduced), it becomes necessary to observe the elastic body 1 at a higher ratio for obtaining high estimation accuracy.

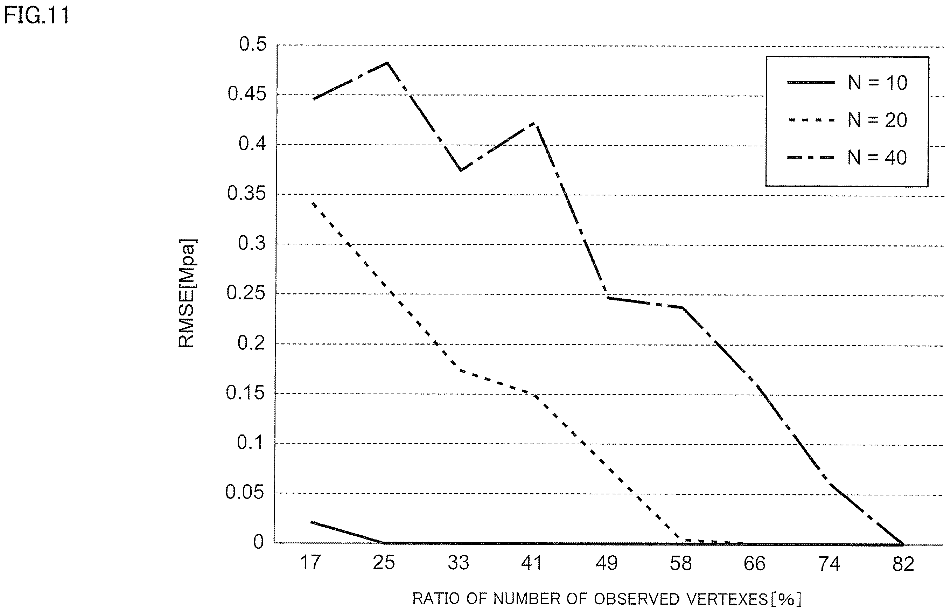

FIG. 11 is a diagram showing experimental results in the cases of the number of estimation blocks N is 10, 20, and 40. The vertical axis in FIG. 11 represents a root mean square error (RMSE) between the true elastic modulus E and the estimated elastic modulus E', and the horizontal axis represents the ratio of the number of observed vertexes accounting for the total (refer to FIG. 9).

Of course, in estimating the elastic modulus E', as described above, the estimation processing for determining the optimal solution is started in the state where the elastic moduli E of the same value are given to all the vertexes of the mesh model 200.

$$\text{RMSE} = \sqrt{\frac{1}{N} \sum_{i=0}^{N-1} \{(E_i - E'_i)^2\}} \qquad \dots \text{Equation 8}$$

According to FIG. 11, when the mesh model 200 is expressed by 10 blocks (when estimation is conducted at low resolution), it can be seen that, even though the number of observed vertexes accounts for about 15% of the entire mesh model 200, the elastic modulus E can be estimated with high accuracy. Moreover, in this case, since the number of blocks is small, the calculation costs can be reduced.

Moreover, when the mesh model 200 is expressed by 20 blocks (when estimation is conducted at medium resolution), it can be seen that, when the number of observed vertexes accounts for about 50% of the entire mesh model 200, the elastic modulus E can be estimated with high accuracy.

When the mesh model 200 is expressed by 40 blocks (when estimation is conducted at high resolution), it can be seen that a least square error is not small enough until the number of observed vertexes accounts for about 70% of the entire mesh model 200. Note that the low resolution, medium resolution and high resolution here merely represent a relative relationship among the three resolutions.

<Brief conclusion>

As described above, according to Exemplary embodiment 1, by determining, as an optimal solution which minimizes the difference $(u_o - u_o')$ between the observed displacement $u_o$ acquired from an image in which a part of the elastic body 1 has been captured and the estimated displacement of a partial region corresponding to an observation region from among estimated displacements $u_o'$ calculated based on the mesh model 200 of the elastic body 1, the magnitude f of the external force F that acts upon an observed manipulation point Pa and a distribution of the elastic modulus E in the entire region of the mesh model 200, it is possible to obtain the distribution of the entire elastic body 1 even when the region where the observed displacement $u_o$ can be acquired is limited to a part of the elastic body 1.

Moreover, the estimation accuracy depends upon the resolution in the estimation space and the ratio of the observed vertexes; if it is desired that the elastic modulus E in the elastic body 1 is estimated at high resolution, the area of the elastic body 1 to be captured by the image-capturing camera 20 is required to be widen, whereas, if it is desired that the elastic modulus E in the elastic body 1 is estimated at low resolution, the area of the elastic body 1 to be captured by the image-capturing camera 20 may be narrow.

<Exemplary embodiment 2>

In the exemplary embodiment, as compared to Exemplary embodiment 1 described above, a method for increasing the estimation accuracy in the distribution of the elastic modulus E will be described.

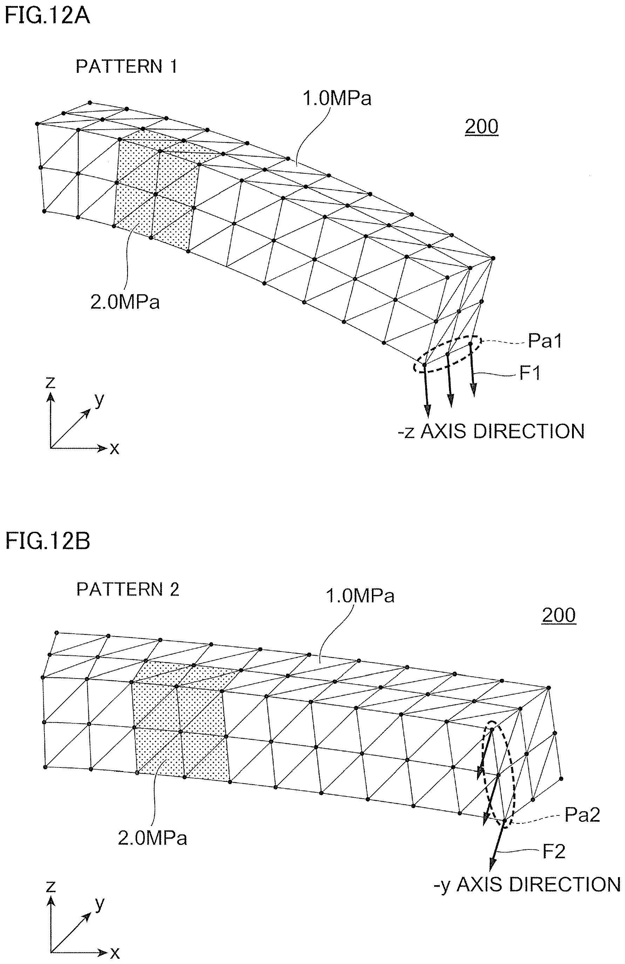

FIG. 12 is a diagram illustrating observation conditions used in Exemplary embodiment 2. In the exemplary embodiment, two sets of external force F and manipulation point Pa are prepared. FIG. 12A shows the first set of external force F1 and manipulation point Pa1 (pattern 1), and FIG. 12B shows the second set of external force F2 and manipulation point Pa2 (pattern 2).

Note that, same as the case of FIG. 8, the mesh model 200 shown in FIG. 12 depicts the cubic block having the elastic modulus E harder than the surroundings by shading.

The first set (pattern 1) shown in FIG. 12A depicts deformation in the case where the first external force F1 is applied in the downward direction (in the -z axis direction) assuming three vertexes at the lower right end part of the mesh model 200 as the manipulation point Pa1. The second set (pattern 2) shown in FIG. 12B depicts deformation in the case where the second external force F2 is applied in the left direction (in the -y axis direction) assuming three vertexes positioned on the left side of the end face at the right end part of the mesh model 200 as the manipulation point Pa2.

Note that, in FIG. 12, the examples in which not only the manipulation point Pa, but also the acting direction of the external force F differs are shown; however, it may be possible that there are one manipulation point Pa and plural acting directions of the external force F, or there are two manipulation points Pa and one acting direction of the external force F.

Also in this case, similar to the case of Exemplary embodiment 1, the information processing device 10 obtains the observed displacement $u_o$ for each pattern, and thereafter, determines, as the optimal solution, the magnitude f1 of the external force F1 and the magnitude f2 of the external force F2 to minimize the difference $(u_o - u_o')$ between the observed displacement $u_o$ and the estimated displacement $u_o'$, and the distribution of the elastic modulus E in the entire region of the mesh model 200 for each pattern.

In other words, the optimization unit 115 determines the unobserved parameter satisfying the following equation (Equation 9) as the optimal solution.

$$E^* = \underset{E,f}{\operatorname{argmin}} J(E, f)$$

$$= \underset{E,f_1,f_2}{\operatorname{argmin}} \{\|u_{1o} - L_o(E)f_1\|_2 + \|u_{2o} - L_o(E)f_2\|_2\}$$

$$= \underset{E,F}{\operatorname{argmin}} \|U_o - L_o(E)F\|_2 \qquad \qquad \ldots \text{Equation 9}$$

Here, $U_o$ is a matrix arranging $u_{1o}$ and $u_{2o}$ in the column direction, and F is a matrix arranging $f_1$ and $f_2$ in the column direction. Moreover, the two-dimensional norm in the equation of the third line is matrix norm, for example, Frobenius norm.

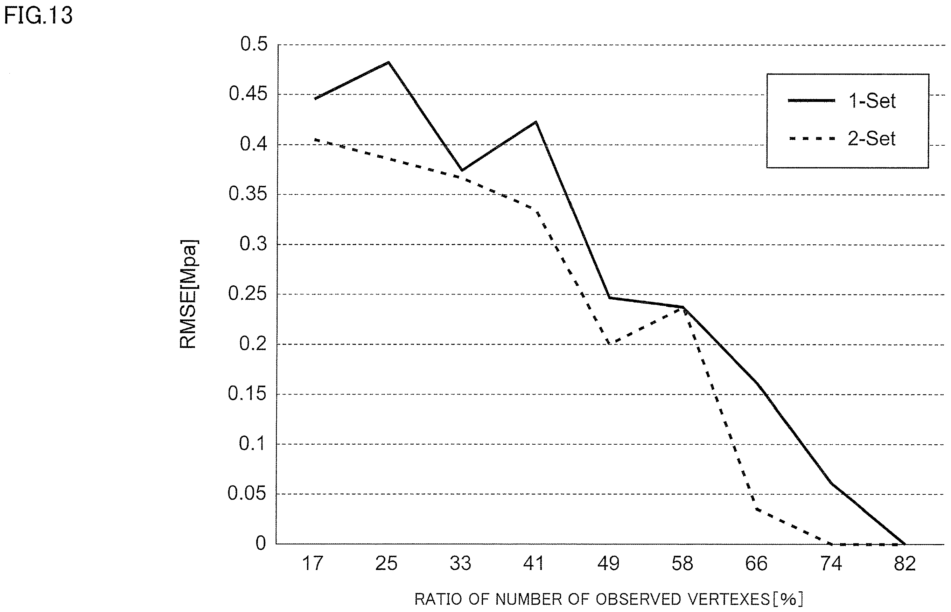

FIG. 13 is a diagram showing experimental results in the cases of using one set (Exemplary embodiment 1) and two sets (Exemplary embodiment 2) of patterns for estimating the distribution of the elastic modulus E. The vertical axis in FIG. 13 represents a root mean square error (RMSE) between the true elastic modulus E and the estimated elastic modulus E', and the horizontal axis represents the ratio of the number of observed vertexes (refer to FIG. 9).

As can be seen from FIG. 13, when the number of sets defined by the manipulation point Pa and the magnitude f of the external force F that are used for estimation of the distribution of the elastic modulus E is "2", as compared to the case of Exemplary embodiment 1 (the case where the number of sets is "1"), the estimation accuracy can be increased even though the ratios of the observed parts are the same.

Note that, in the exemplary embodiment, the number of sets defined by the manipulation point Pa and the magnitude f of the external force F is assumed to be "2"; however, the number of sets may be three or more. In general, as the number of sets is increased, the estimation accuracy can be increased. For example, in the elastography method, which is an example of the method for performing non-invasive evaluation of a strain image generated in an evaluation target (for example, an organ) by application of vibrations, such as ultrasonic waves, the external force F made to be applied to the evaluation target temporally changes. In other words, in the elastography method, there are plural sets defined by the manipulation point Pa and the magnitude f of the external force F. Consequently, the exemplary embodiment is well matched with the elastography method, which makes it possible to perform estimation with high accuracy.

<Exemplary embodiment 3>

Also, in the exemplary embodiment, as compared to Exemplary embodiment 1 described above, another method for increasing the estimation accuracy in the distribution of the elastic modulus E will be described.

Here, description will be given of a case in which sparsity can be estimated in the change of the elastic modulus E. To have sparsity means a property in which most of the elements are 0 (zero). Specifically, the method can be adopted for an elastic body 1 that is known to have localization with partial stiffness or softness. For example, in an organ, whereas the elastic modulus E in a healthy tissue is uniform, it is assumed that only part of a tumor region is hard (the elastic modulus E is high) as compared to the healthy tissue. Consequently, the sparsity can be utilized for estimating the distribution of the elastic modulus E in an organ.

In the optimization problem, by introducing a sparse term to the change of the elastic modulus E, it is possible to place a constraint on the estimated value of the elastic modulus E to change locally, and thereby improvement of the estimation accuracy can be expected.

In the exemplary embodiment, the optimization unit 115 determines the unobserved parameter satisfying the following equation (Equation 10) as the optimal solution.

$$E^* = \underset{E,f}{\arg\min} J(E, f) = \underset{E,f}{\arg\min} \{ \|u_o - L_o(E)f\|_2 + \lambda \|E - E_0\|_1 \} \qquad \text{... Equation 10}$$

The second term in Equation 10 is the sparse term. Here, Eo is a reference value of the elastic modulus applied to most of the vertexes. Consequently, the sparse term is an expression that determines a one-dimensional norm $L^1$ for a difference between the elastic modulus E estimated for each vertex and the reference value (that is, $\Delta E$).

In the exemplary embodiment, the coefficient $\lambda$ was assumed to be 0.1. In the case of the expression, with respect to only partial vertexes with the elastic moduli E different from the reference value, the difference $\Delta E$ (= $E - E_0$) is non-0 (non-zero), and the difference $\Delta E$ in most of the vertexes is 0 (zero). In the sparse term, when the distribution of the elastic moduli E is highly localized, the norm is reduced. Consequently, the distribution of the elastic modulus E with a large norm in the sparse term is evaluated low. Note that the value of the coefficient $\lambda$ is a specific example.

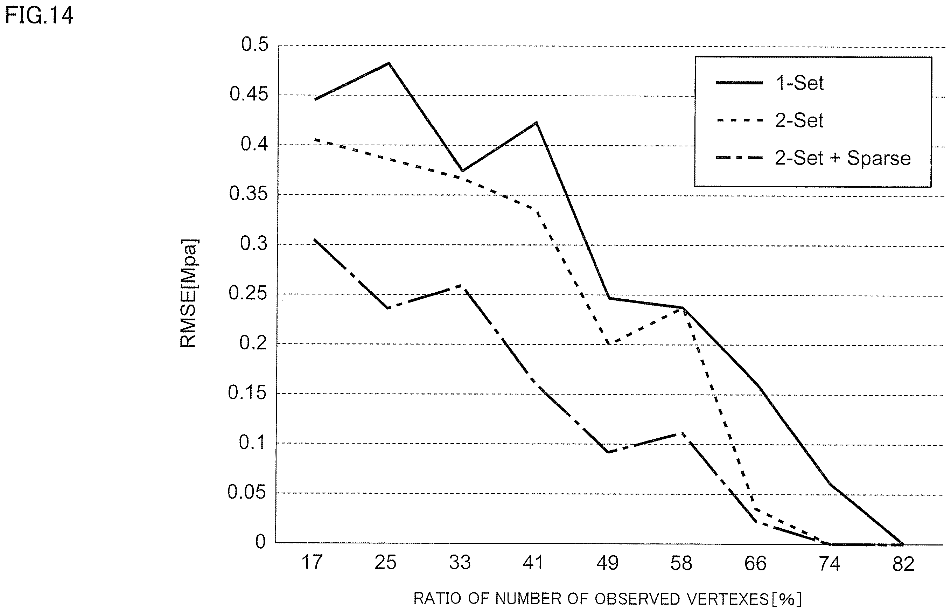

FIG. 14 is a diagram showing experimental results in the cases of using one set of patterns (Exemplary embodiment 1) and two sets (Exemplary embodiment 2) of patterns for estimating the distribution of the elastic modulus E, and using sparsity of changes in the elastic modulus E (Exemplary embodiment 3) in addition to the case of two sets of patterns. The vertical axis in FIG. 14 represents a root mean square error (RMSE) between the true elastic modulus E and the estimated elastic modulus E', and the horizontal axis represents the ratio of the number of observed vertexes (refer to FIG. 9).

As can be seen from FIG. 14, when the sparsity in the change of the elastic modulus E is combined with Exemplary embodiment 2, it is leaned that the estimation accuracy is increased as compared to any of the case of Exemplary embodiment 1 (when the number of sets is "1") and the case of Exemplary embodiment 2 (when the number of sets is "2"). In particular, when the sparsity in the change of the elastic modulus E is combined with Exemplary embodiment 2, it is possible to obtain high estimation accuracy even in the case where the ratio of the number of observed vertexes is low.

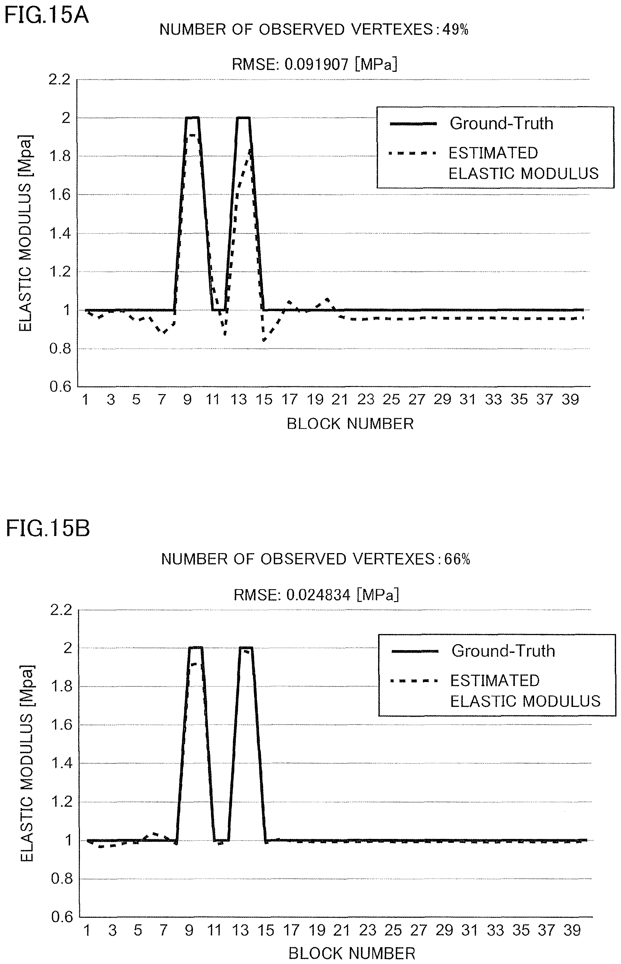

FIG. 15 is a diagram illustrating a relationship between the ratio of the number of observed vertexes and the observation accuracy in the exemplary embodiment. In FIG. 15, the vertical axis indicates the elastic modulus, and the horizontal axis indicates the block number. FIG. 15 represents the case in which the number of estimation blocks N is 40. In the figure, the change of the true elastic modulus E is represented by a rectangular pulse, and the change of the estimated elastic modulus E is represented by a broken line.

As shown in FIG. 15A, when the number of the observed vertexes accounts for 49% of the total, two waveforms almost match with each other, whereas, as shown in FIG. 15B, when the number of observed vertexes accounts for 66% of the total, the overlapping degree of the two waveforms is further increased. Consequently, in the case of the observation conditions that are preconditioned in FIG. 15, it is learned that, by observing about 50% of the elastic body 1, which is the observation object, it is possible to roughly estimate which region is hard (RMSE = 0.091907 MPa), and by observing about 60% or more, it is possible to estimate which region is hard with higher accuracy (RMSE = 0.024834 MPa).

<Other exemplary embodiments>

In the above-described exemplary embodiments, description was given of the case in which any of the magnitude f of the external force F and the distribution of the elastic modulus E was unobserved; however, when the magnitude f of the external force F is observed, since the number of unobserved parameters is reduced, the estimation accuracy of the distribution of the elastic modulus E can be increased as compared to Exemplary embodiment 1.

Each of the above-described exemplary embodiments can be utilized for various purposes. For example, while the information processing system 100 can be utilized for the medical support system that supports diagnosis or surgery by a doctor, the system 100 can be utilized in industrial fields other than the medical field, for purposes of observation or analysis, and for purposes of production and manufacturing.

For example, when the information processing system 100 is utilized as the medical support system, as described above, even though it is impossible to observe the entire image of an organ at once because of the limited view, the distribution of the elastic modulus E that is unique to an organ of a patient, who is an object under study, can be estimated with high accuracy by use of information about changes of the organ occurred in the observed region (the observed displacement $u_o$).

In the aforementioned exemplary embodiments, description was given on the assumption that a still image was taken by the image-capturing camera 20; however, the image-capturing camera 20 may be an image-capturing unit capable of capturing the elastic body 1, which is the estimation target of the elastic modulus E, as a dynamic image. In this case, the above-described observed displacement $u_o$ may be acquired by use of a still image captured from the dynamic image that has been taken.

Moreover, in the aforementioned exemplary embodiments, description was given of the case of a single image-capturing camera 20; however, there may be plural image-capturing cameras 20. In addition, the image capturing direction is not limited to one, but plural image capturing directions may be adopted. The information about the observed displacement $u_o$ can also be increased by using images capturing the deformation by the external force F from the plural directions, and thereby the estimation accuracy can be improved.

Moreover, in the above-described exemplary embodiments (for example, in Exemplary embodiment 1), description was given on the assumption that the number of estimation blocks N was provided as a fixed value; however, it may be possible to adopt the method for gradually increasing the spatial resolution of the estimation result while increasing the number of estimation blocks N. In other words, the estimation processing of the distribution of the elastic modulus E may be divided into plural steps.

For example, it may be possible that, in the initial estimation step, the estimation processing is executed for the case in which the number of estimation blocks N is 10, to thereby obtain the estimation result in a short time and display thereof on the display device 40, and in the next estimation step, the number of estimation blocks N is increased to the predetermined value (for example, 20) and an update of execution of the estimation processing and display of the estimation result is repeated. According to such a method, a user can acquire, while grasping a rough image of the distribution of the elastic modulus E early, a higher spatial resolution as time passes.

So far, the exemplary embodiment according to the present invention has been described, but the technical scope of the present invention is not limited to the scope described in the above-described exemplary embodiment. It is obvious from the following claims that various modifications and improvements added to the above-described exemplary embodiment are also included in the technical scope of the present invention.

Reference Signs List

| | |
|---|---|
| 1: | Elastic body |
| 10: | Information processing device |
| 100: | Information processing system |
| 111: | Mesh model generation unit |
| 112: | Observed displacement acquisition unit |
| 113: | Manipulation point acceptance unit |
| 114: | Displacement estimation unit |
| 115: | Optimization unit |
| 116: | Expression unit |
| 200: | Mesh model |
| E: | Elastic modulus |
| F: | External force |
| f: | Magnitude of external force |

$u_o$:     Observed displacement
$u_o$':    Estimated displacement
Pa:     Manipulation point
Pf:     Fixed point

**Claims**

1.  An information processing device comprising:
    a computation unit that determines, as an optimal solution which minimizes a difference between an observed displacement having been acquired from an image in which a portion of an elastic body has been captured and an estimated displacement of a partial region corresponding to an observation region from among estimated displacements calculated based on a three-dimensional model of the elastic body, a magnitude of an external force that acts upon an observed manipulation point and a distribution of an elastic modulus in the three-dimensional model.

2.  The information processing device according to claim 1, wherein the computation unit determines the magnitude of the external force and the distribution of the elastic modulus based on an equation as follows:

$$E^* = \underset{E,f}{\mathrm{argmin}}\, J(E,f) = \underset{E,f}{\mathrm{argmin}}\{\|u_o - L_o(E)f\|_2 + \lambda\|E - E_0\|_1\}$$

    where E* represents a set of E and f that minimizes an evaluation function J (E, f);
    E represents an elastic modulus;
    Eo represents a reference value of the elastic modulus;
    f represents a magnitude of an external force;
    $u_o$ represents an observed displacement;
    $L_o(E)$ represents an observed part of an inverse matrix component of a total stiffness matrix K expressed by the elastic modulus E;
    $L_o(E)f$ represents an estimated displacement;
    $\lambda$ represents a coefficient;
    $\| \ \ \|_1$ represents a one-dimensional norm; and
    $\| \ \ \|_2$ represents a two-dimensional norm.

3.  An information processing device comprising:

    an acquisition unit that acquires an observed part of displacement from an image of an elastic body having been captured;
    an acceptance unit that accepts a manipulation point of an external force acting upon the elastic body;
    an estimation unit that estimates an estimated displacement of a three-dimensional model of the elastic body by using a distribution of an elastic modulus in the three-dimensional model and a magnitude of the external force as variables; and
    an optimization unit that minimizes a difference between the observed displacement and the estimated displacement corresponding to the observed displacement, so as to find an optimal solution that determines the magnitude of the external force and the distribution of the elastic modulus in the three-dimensional model.

4.  The information processing device according to claim 3, wherein the optimization unit determines the magnitude of the external force and the distribution of the elastic modulus based on an equation as follows:

$$E^* = \underset{E,f}{\mathrm{argmin}}\, J(E,f) = \underset{E,f}{\mathrm{argmin}}\{\|u_o - L_o(E)f\|_2 + \lambda\|E - E_0\|_1\}$$

    where E* represents a set of E and f that minimizes an evaluation function J (E, f);
    E represents an elastic modulus;
    Eo represents a reference value of the elastic modulus;
    f represents a magnitude of an external force;
    $u_o$ represents an observed displacement;

$L_o(E)$ represents an observed part of an inverse matrix component of a total stiffness matrix K expressed by the elastic modulus E;
$L_o(E)f$ represents an estimated displacement;
$\lambda$ represents a coefficient;
$||\,|\,|_1$ represents a one-dimensional norm; and
$||\,|\,|_2$ represents a two-dimensional norm.

**5.** The information processing device according to claim 1 or 3, further comprising:
an expression unit that expresses the three-dimensional model based on the distribution of the elastic modulus determined as the optimal solution.

**6.** The information processing device according to claim 1 or 3, wherein the image is a still image captured by a single image-capturing camera.

**7.** The information processing device according to claim 1 or 3, wherein the optimal solution is determined as a value that minimizes a sum total of a sum of absolute values of changings from an initial value of the distribution of the elastic modulus and the difference.

**8.** The information processing device according to claim 1 or 3, wherein, when a plurality of pieces of information about the manipulation point is provided, the optimal solution is determined as a value that minimizes a sum total of the differences calculated for the individual manipulation points.

**9.** The information processing device according to claim 1 or 3, wherein, when a plurality of the external forces is applied to the manipulation point, the optimal solution is determined as a value that minimizes a sum total of the differences calculated for the individual external forces.

**10.** The information processing device according to claim 1 or 3, wherein there is a plurality of the manipulation points, and the external forces, which are different from one another, are applied to the respective manipulation points.

**11.** The information processing device according to claim 1 or 3, wherein a spatial resolution that determines the optimal solution is gradually increased from a low level.

**12.** The information processing device according to claim 1 or 3, wherein, when a magnitude of the external force that acts upon the manipulation point is given, the optimal solution is determined as the distribution of the elastic modulus that minimizes the difference.

**13.** An information processing method comprising:

a process that acquires an observed part of displacement from an image of an elastic body having been captured;
a process that accepts a manipulation point of an external force acting upon the elastic body;
a process that estimates an estimated displacement of a three-dimensional model of the elastic body by using a distribution of an elastic modulus in the three-dimensional model and a magnitude of the external force as variables; and
a process that minimizes a difference between the observed displacement and the estimated displacement corresponding to the observed displacement, so as to find an optimal solution that determines the magnitude of the external force and the distribution of the elastic modulus in the three-dimensional model.

**14.** A program causing a computer to execute:

a process that acquires an observed part of displacement from an image of an elastic body having been captured;
a process that accepts a manipulation point of an external force acting upon the elastic body;
a process that estimates an estimated displacement of a three-dimensional model of the elastic body by using a distribution of an elastic modulus in the three-dimensional model and a magnitude of the external force as variables; and
a process that minimizes a difference between the observed displacement and the estimated displacement corresponding to the observed displacement, so as to find an optimal solution that determines the magnitude of the external force and the distribution of the elastic modulus in the three-dimensional model.

# FIG.1

UNOBSERVED PART   OBSERVED PART

ESTIMATE DISTRIBUTION OF ELASTIC MODULUS
IN ENTIRETY OF ELASTIC BODY
INCLUDING UNOBSERVED PART

FIG.2

IMAGE-CAPTURING
CAMERA

20

10

INFORMATION
PROCESSING
DEVICE

40

DISPLAY
DEVICE

11

CPU

12

ROM

13

RAM

14

STORAGE
UNIT

30

THREE-DIMENSIONAL
IMAGE DATA
STORAGE DEVICE

50

INPUT
DEVICE

100

FIG.3

10

THREE-DIMENSIONAL IMAGE DATA

111 — MESH MODEL
GENERATION UNIT

THREE-DIMENSIONAL
MODEL

MANIPULATION POINT

113

MANIPULATION POINT
ACCEPTANCE UNIT

CAMERA
IMAGE

OBSERVED
DISPLACEMENT
ACQUISITION UNIT

112

MANIPULATION POINT
INFORMATION

114

DISPLACEMENT
ESTIMATION
UNIT

OBSERVED
DISPLACEMENT $u_0$

ESTIMATED
DISPLACEMENT $u_0'$

115

E AND $f$

OPTIMIZATION UNIT
(UPDATE OF E and $f$)

DISTRIBUTION OF
ELASTIC MODULUS

EXPRESSION UNIT — 116

TO 40

FIG.4

200

FIG.5

EP 3 564 901 A1

FIG.6

ELEMENT I ⇒ CONSTITUTED BY 1, 2 and 3

ELEMENT II ⇒ CONSTITUTED BY 2, 3 and 4

# FIG.7

K

$$
\begin{array}{|c|c|c|c|}
\hline
K_{11} & K_{12} & K_{13} & K_{14} \\
\hline
K_{21} & K_{22} & K_{23} & K_{24} \\
\hline
K_{31} & K_{32} & K_{33} & K_{34} \\
\hline
K_{41} & K_{42} & K_{43} & K_{44} \\
\hline
\end{array}
$$

=

Ke OF ELEMENT I

$$
\begin{array}{|c|c|c|c|}
\hline
K_{11} & K_{12} & K_{13} & K_{14} \\
\hline
K_{21} & K_{22} & K_{23} & K_{24} \\
\hline
K_{31} & K_{32} & K_{33} & K_{34} \\
\hline
K_{41} & K_{42} & K_{43} & K_{44} \\
\hline
\end{array}
$$

+

Ke OF ELEMENT II

$$
\begin{array}{|c|c|c|c|}
\hline
K_{11} & K_{12} & K_{13} & K_{14} \\
\hline
K_{21} & K_{22} & K_{23} & K_{24} \\
\hline
K_{31} & K_{32} & K_{33} & K_{34} \\
\hline
K_{41} & K_{42} & K_{43} & K_{44} \\
\hline
\end{array}
$$

FIG.8

1.0MPa

2.0MPa

200

EP 3 564 901 A1

FIG.9

FIG.10

N = 10

N = 20

N = 40

EP 3 564 901 A1

FIG.11

# FIG.12A

PATTERN 1

1.0MPa

200

2.0MPa

z
y
x

Pa1

F1

-z AXIS DIRECTION

# FIG.12B

PATTERN 2

1.0MPa

200

2.0MPa

z
y
x

Pa2

F2

-y AXIS DIRECTION

FIG.13

RATIO OF NUMBER OF OBSERVED VERTEXES[%]

EP 3 564 901 A1

FIG.14

EP 3 564 901 A1

FIG.15A

NUMBER OF OBSERVED VERTEXES:49%

FIG.15B

NUMBER OF OBSERVED VERTEXES:66%

## INTERNATIONAL SEARCH REPORT

International application No.

PCT/JP2017/044527

A.   CLASSIFICATION OF SUBJECT MATTER
Int. Cl.  G06T7/00(2017.01)i, A61B1/045(2006.01)i, A61B8/08(2006.01)i

According to International Patent Classification (IPC) or to both national classification and IPC

B.   FIELDS SEARCHED

Minimum documentation searched (classification system followed by classification symbols)
Int. Cl.  G06T7/00, A61B1/045, A61B8/08

Documentation searched other than minimum documentation to the extent that such documents are included in the fields searched
Published examined utility model applications of Japan    1922-1996
Published unexamined utility model applications of Japan  1971-2018
Registered utility model specifications of Japan          1996-2018
Published registered utility model applications of Japan  1994-2018

Electronic data base consulted during the international search (name of data base and, where practicable, search terms used)

C.   DOCUMENTS CONSIDERED TO BE RELEVANT

| Category* | Citation of document, with indication, where appropriate, of the relevant passages | Relevant to claim No. |
|---|---|---|
| Y<br>A | JP 2011-177535 A (GEN HOSPITAL CORP) 15 September 2011, paragraphs [0031]-[0034], [0038]-[0048], [0104] & US 2006/0058592 A1, paragraphs [0043]-[0046], [0050]-[0055], [0091] & WO 2006/024014 A2 & EP 1989997 A1 | 1, 5-12<br>2-4, 13-14 |
| Y<br>A | JP 2015-97724 A (TOSHIBA CORP.) 28 May 2015, paragraphs [0040]-[0047], [0066]-[0067] (Family: none) | 1, 5-12<br>2-4, 13-14 |

☐   Further documents are listed in the continuation of Box C.          ☐   See patent family annex.

| | |
|---|---|
| *        Special categories of cited documents:<br>"A"    document defining the general state of the art which is not considered to be of particular relevance<br>"E"    earlier application or patent but published on or after the international filing date<br>"L"    document which may throw doubts on priority claim(s) or which is cited to establish the publication date of another citation or other special reason (as specified)<br>"O"    document referring to an oral disclosure, use, exhibition or other means<br>"P"    document published prior to the international filing date but later than the priority date claimed | "T"    later document published after the international filing date or priority date and not in conflict with the application but cited to understand the principle or theory underlying the invention<br>"X"    document of particular relevance; the claimed invention cannot be considered novel or cannot be considered to involve an inventive step when the document is taken alone<br>"Y"    document of particular relevance; the claimed invention cannot be considered to involve an inventive step when the document is combined with one or more other such documents, such combination being obvious to a person skilled in the art<br>"&"    document member of the same patent family |

| Date of the actual completion of the international search | Date of mailing of the international search report |
|---|---|
| | |

| Name and mailing address of the ISA/<br>    Japan Patent Office<br>    3-4-3, Kasumigaseki, Chiyoda-ku,<br>    Tokyo 100-8915, Japan | Authorized officer<br><br>Telephone No. |

Form PCT/ISA/210 (second sheet) (January 2015)

32

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **O. GOKSEL ; H. ESKANDARI ; S. E. SALCUDEAN.** Mesh adaptation for improving elasticity reconstruction using the FEM inverse problem. *IEEE Trans. Med. Imaging,* 2013, vol. 32 (2), 408-418